(19) Europäisches Patentamt

European Patent Office

Office européen des brevets

(11) Publication number : **0 490 600 A1**

# (12) EUROPEAN PATENT APPLICATION

(21) Application number : **91311403.9**

(22) Date of filing : **06.12.91**

(51) Int. Cl.⁵ : **A01H 4/00,** A01H 5/10

The microorganism(s) has (have) been deposited with American Type Culture Collection under number(s) 75136.

(30) Priority : **06.12.90 US 622982**

(43) Date of publication of application :
**17.06.92 Bulletin 92/25**

(84) Designated Contracting States :
**AT BE CH DE DK ES FR GB GR IT LI LU NL SE**

(71) Applicant : **PIONEER HI-BRED INTERNATIONAL, INC.
700 Capital Square 400 Locust Street
Des Moines Iowa 50309 (US)**

(72) Inventor : **Bidney, Dennis
8385 Plum Drive, Des Moines
Polk County, Iowa 50323 (US)**
Inventor : **Burrus, Monique
7, Rue Du Beau-Regard
F-67220 Ville (FR)**
Inventor : **Garnaat, Carl W.
401 NE Innsbruck, Ankeny
Polk County, Iowa 50021 (US)**
Inventor : **Heaton, Tom
909 Alvardado Avenue No.36, Davis
Yolo County, CA 95616 (US)**

(74) Representative : **Goldin, Douglas Michael et al
J.A. KEMP & CO. 14, South Square Gray's Inn
London WC1R 5EU (GB)**

(54) Sunflower plants having a high frequency of regenerability of fertile plants from isolated protoplasts and method of usingsame.

(57)   A sunflower variety designated PTO24 has been found to have a very high capability of regenerating fertile plants from single protoplasts. A method of using said variety to regenerate fertile sunflower plants and to impart the regenerative trait to other, non-regenerating varieties is also disclosed.

EP 0 490 600 A1

## Background of the Invention

The sunflower, Helianthus annuus L., is one of the world's most important agricultural crops. Sunflower is grown for ornamental purposes and sunflower seeds are produced for animal and human consumption. Sunflower is one of the four major annual crops grown for edible oil. Approximately 90% of all sunflower grown in the United States is used in oil production.

The ability to genetically transform sunflower plants would be of great benefit to the development of agronomically superior sunflower varieties. For example, genetic transformation could be used to develop sunflower varieties that are resistant to diseases or insects or that have improved nutritional qualities.

One of the key steps to achieving genetically transformed sunflower plants is the ability to regenerate fertile plants from single protoplasts. When a single, transformed protoplast is regenerated into a plant, the transferred DNA is present in every cell of the plant, including the germ cells. If this transformed plant produces fertile seed, the plants resulting from those seeds will also have the new DNA.

The transformation of isolated plant protoplasts using naked DNA has become a routine procedure. However, one major limitation in the application of protoplast transformation technology to crop plant improvement efforts is the ability to recover fertile plants from protoplast derived calli following DNA transfer into protoplasts. While a number of plants have been regenerated from protoplasts, including representatives of the genera Chicorum, Chrysanthemum, Crepis, Dimorphotheca, Lactiva, Petasites, Rudbeckia, Senecio, and Gallardia. Roest, S. and Gilissen, L.J.W., 38(1) Acta Bot. Neerl. 1 (1989), sunflower has proven to be a difficult species to regenerate from protoplasts. At the same time, reports have been published describing regeneration of plants from callus of hypocotyl explants of germinating seeds, Paterson, K.E. and Everett, N.P., 42 Plant Science 125 (1986); Greco, B. et al, 36 Plant Science 73 (1984); immature embryos, Finer J.J. 6 Plant Cell Reports 372 (1987); Power, C., 71(7) Amer. J. Bot. 497 (1987); Freyssinet M. and Freyssinet G. 56 Plant Science 177 (1988); shoot tips, Paterson K. E., 71(7) Amer. J. Bot. 925 (1984); Bohorova N. E. et al. 95 Z. Pflanzenphysiol. 119 (1985); Lupi M.C. et al. 11 Plant Cell Tissue and Organ Culture 47(1987); and stem parenchyma, Bohorova N.E. et al., supra.

The first report of shoot regeneration from protoplast derived calli of sunflower was by Binding, H. et al., 101 Z. Pflanzenphysiol. 119 (1981), where organization was observed from shoot apex protoplast calli on a 85 medium supplemented with 15μM BAP. But apart from the reported observation of in vitro flowering, no information was presented concerning reproducibility of the system and whether it was possible to recover fertile plants under greenhouse conditions. Subsequent attempts to develop a protoplast-to-plant regeneration system in sunflower were reported to be unsuccessful for hypocotyl protoplasts, Lenee P. and Chupeau Y., 43 Plant Science 69 (1986); Bohorova N.E. et al., 5 Plant Cell Reports 256 (1986); Dupuis J.M. et al., 307 C.R. Acad. Sci. Paris 465 (1988); Moyne A.L. et al., 7 Plant Cell Reports 437 (1988); Chanabe C. et al., 64 Plant Science 125 (1989); mesophyll or cotyledonary protoplasts, Bohorova, supra, (1986); Schmitz P. and Schnabl H., 135 J. Plant Physiol. 223,(1989); Guilley E. and Hahne. G., 8 Plant Cell Reports 225 (1989). To date, no sunflower germplasm has been reported to be regenerable to whole plants from protoplasts.

It is well documented that genetic variability affects the behavior of plant cells in culture for members of the families Solanaceae, Leguminosae, and Graminaeae. See K.E. Paterson and N.P. Everett, 42 Plant Science 125 (1985); G.J. Keyro, G.B. Collins and N. L. Taylor, 58 Theor. Appl. Genet. 265 (1980); M. F. Deitert, S. A. Barron, and O. E. Yoder, 26 Plant Sci.Litt. 265 (1982). The variation between cultivars has been demonstrated to be as great as that between species. Paterson, et al, supra and Deitert, et al., supra. The disclosures of the foregoing articles are incorporated herein by reference to show the state of the art.

In order to increase the probability of obtaining transgenic plants using other transformation methods such as Agrobacterium, it is also desirable to use plants which have a high frequency of regeneration of fertile plants from tissue culture. However, in the past,such plants have not routinely been available in sunflower research. Accordingly, a continuing need exists for sunflower varieties which exhibit heritable, consistent, high levels of regenerability to fertile plants from protoplast tissue culture.

An object of this invention is a sunflower line which consistently gives a very high frequency of regeneration from hypocotyl explants in tissue culture and is also capable of regenerating fertile plants from cultured protoplasts. A second object of this invention is a method of regenerating sunflower plants from material obtained from such a line. Yet another object of this invention is to provide a substantially homozygous inbred sunflower line which can be used directly in research or as a source of the regenerability trait.

## Detailed Description of the Invention

This invention provides a sunflower plant having a frequency of regeneration of fertile plants of from about 80% to 100% from hypocotyl explants and the capability to regenerate fertile plants from hypocotyl protoplasts in tissue culture, and seed thereof. Various parts of the plant such as leaves, pollen, embryos, protoplasts, meristematic cells, hypocotyls, petals, seeds, stalks, and tissue cultures of those parts are useful for research purposes and the seeds are useful for commercial purposes, such as wax and oil production end bird and animal feed. An important use of the plants of this invention is as donors of the trait for regenerability to commercial sunflower germplasm for use in plant breeding research.

Hypocotyl explants from 11 day old sunflower seedlings were plated on medium R501 as defined hereinafter. The explants were cultured in the dark for two weeks and then placed in the light. Regeneration was scored after 1 week in the light.

Regeneration frequencies were scored by counting the number of plants which had at least one explant with regeneration compared to the total number of plants screened. Also for some of the lines, the frequency of explants which showed regeneration compared to the total number of explants was scored.

Initial screening showed that seed from a selection out of the United States Department of Agriculture's germplasm release SFM3 had potential for high frequency of regeneration when compared to other sunflower genotypes. However, the frequency of regeneration within this selection has been highly variable and inconsistent. The regenerants from these seedlings were selected, grown to maturity, and the mature plants were self pollinated to produce seed. The progeny of one selection has consistently exhibited a very high frequency of regeneration from hypocotyl explants. This high frequency of regeneration has been maintained in three self-pollinated generations of plants. Protoplasts from this line have also been regenerated to whole plants, and although the frequency of such regeneration is low, no other sunflower germplasm has been reported to be regenerable to whole plants from protoplasts.

Accordingly, the present invention provides a sunflower line obtained from USDA germplasm release SFM3 by first selecting a 1-tall-2 genotype out of the release, and then selecting individual seedlings of that genotype that exhibited a high frequency of regeneration ability, and self pollinating the regenerates of those seedlings. The process is continued for at least two generations of cycling from whole plants, to tissue culture from the plants, to regeneration of plants from the tissue culture, to self-pollination of the regenerated plants, to planting of the seed obtained from the self-pollinated plants, to growth of whole plants from the seed, and back to tissue culture. Thus, the line is a second generation self from the 1-tall-2 selection from a fourth generation self of a male fertile segregant from a cross of cytoplasmic male sterile HA89 with pollen from Helianthus petiolaris and backcrossed once to cytoplasmic male sterile line HA89; having a frequency of regeneration to whole fertile plants from hypocotyl explants in tissue culture of from about 80% to about 100%; and the ability to regenerate whole fertile plants from hypocotyl protoplasts.

SFM3 is a $S_4$ single plant selection derived from a cmsHA89/Helianthus petiolaris Nuttall//cmsHA89 backcross. As a fourth generation self, SFM3 is regarded by breeders as a "germplasm" release, i.e. with over 3% off-type alleles, it still contains a significant amount of genetic variability as compared to an "inbred" release which has six or more generations of selfing and selection for type. The release was intended by USDA to provide germplasm to sunflower breeders that could be used to infer resistance to Sunflower Moth larva. Two generations of selfing and selection out of SFM3 yielded the proprietary line SFM3 1-TALL-2. The 1-tall-2 genotype was then selected for regenerability over an additional two generations of tissue culture, regeneration, selfing, and re-culturing of explants to yield inbred sunflower line PTO24. PTO24, being substantially homozygous over all loci, carries the trait for regenerability in a genetic form which is suitable for providing this trait to the progeny of a second cross (first backcross) in a breeding program using conventional cross-and backcross methods.

The comparison of regeneration frequency of SFM3 1-tall-2 and progeny, including PTO24, is shown in the following table.

## Comparison of Regeneration Frequencies of
## SFM3 1-tall-2 and Progeny

| Genotype | #regen plant /total plants | % | #regen explants /total explants | % |
|---|---|---|---|---|
| SFM3 1-tall-2 | 80/155 | 52% | | |
| PT001-R$_1$ (3-26-87-E) | 1/1 | | | |
| PT003-R$_1$ (3-26-87-E) | 2/3 | 67 | | |
| PT016-R$_1$ (2-11-87-S$_3$) | 2/3 | 67 | | |
| PT019-R$_1$ (2-11-87-S$_3$) | 5/7 | 71 | 24/76 | 32% |
| PT021-R$_1$ (2-25-87-E) | 8/8 | 100 | 52/73 | 71 |
| PT021-R$_2$ (2-25-87-E) | 4/5 | 80 | 14/31 | 45 |
| PT024-R$_1$ (2-6-87-X$_1$) | 34/34 | 100 | 196/204 | 96 |
| PT024-R$_2$ (2-6-87-X$_1$) | 5/5 | 100 | 25/25 | 100 |

**Variety Description - PTO24**

PTO24 is an inbred variety of annual sunflower, Helianthus annus L, that is best described as follows: PTO24 is 116 cm. tall at harvest ripeness and has a 5 cm. length of internode. It produces a visible head 32 days from emergence and matures at 115 days. The leaves of PTO24 are approximately 17.5 cm. in length and 20 cm. in width. The leaves are serrate and green in color. The head at flowering has yellow ray flowers and purple disk flowers. The ray length is approximately 75mm. and the width is approximately 20mm. At maturity, the seed head is 17.5cm. in diameter and contains approximately 840 seeds per head. The seeds are obovate in shape and nearly black in color. A more detailed description of PTO24 is provided in the table below. PTO24 most closely resembles the variety HA89, a publicly released variety developed by the Agriculture Research Service, United States Department of Agriculture. However, HA89 does not exhibit the regeneration capabilities of PTO24.

## PTO24
## Variety Description Information

Comparison Variety: HA89

A.  Class:                                                              Oil Type

B.  Maturity:

    No. of days to head first visible:     32
    Days earlier than HA89:     1

    No. of days to harvest ripeness:     115
    Days earlier than HA89:     1

C.  Height:

    Tall at harvest ripeness:     116 cm
    Taller than HA89:     36 cm

D.  Stem:

    Length of internode at harvest ripeness:     5 cm
    Branching:     No branching
    Color of growing point:     Green)
    Number of leaves:     34
    More leaves than HA89:     8

E.  Leaves:

    Blade length:     17.5 cm

    Blade width:     20.0 cm
    Shorter than HA89:     5 cm

Narrower than HA89:                                           4 cm
Width:                                             Wider than long
Leaf Shape:                                               Cordate
Leaf Apex:                                              Acuminate
Leaf Margin:                                              Serrate
Depth of Margin Indentations:                            Shallow
Attitude:                                             Descending
Surface:                                                  Smooth
Color:                                                     Green
Margin Color:                                             Green

F.    Head at Flowering:

Ray Flowers:                                             Present
Ray Flower Color:                                         Yellow
Disk Flower Color:                                        Purple
Anthocyanin in Stigmas:                                 Present
Pollen Color:                                            Yellow
Pappi:                                                    Green
Ray Length:                                               75 mm
Shorter than HA89:                                        5 mm
Ray Width:                                               20 mm

G.    Head at Seed Maturity:

Diameter:                                               17.5 cm
Receptable Shape:                                       Concave
Head Attitude:                                         Ascending
No. of seeds per head:          - 840 (Poor self-fertility)
Seeds/head less than HA89:                                 800

H.    Seeds:

Outer Pericarp:              Nearly solid black/purplish
Middle Pericarp:                            Solid Purple

| Inner Pericarp: | No color |
| Stripes: | Absent |
| Mottling: | Absent |
| Shape: | Obovate |
| Length: | 10.0 mm |
| 100 seed: | 8.0 g |
| % held on 7.9 mm (20/64) round-hold screen: | 0.0 |

I.   Disease and Insects:

| Rust: | Not tested |
| Verticillium Wilt: | Not tested |
| Downy Mildew: | Not tested |
| White Blister Rust: | Not tested |
| Broom Rape: | Not tested |
| European Sunflower Moth: | Not tested |
| Sclerotinia Wilt: | Not tested |
| Leaf Mottle: | Not tested |
| Gray-Mold Blight, Bud Rot: | Not tested |
| Charcoal Rot, Stem Rot: | Not tested |
| Sunflower Moth: | Resistant |

Because sunflower line PT024 is substantially homozygous, it can be reproduced by planting seeds of the line, growing the resulting sunflower plants under self pollinating or sib-pollinating conditions with adequate isolation, and harvesting the resulting seed, using techniques familiar to the agricultural arts. This variety can be used directly in research or commercial sunflower seed production, or as a source of the homozygous trait for regenerability in a cross to another, different sunflower line which is to be the "target" of a transformation process. Conventional plant breeding methods can be used to transfer the regenerability trait provided by this variety to any desired "target" variety via crossing and backcrossing. Such methods will comprise the steps of:

a) sexually crossing a plant of this invention with a plant from the target variety;
b) recovering reproductive material from the progeny of the cross;
c) selfing the progeny of the cross;
d) screening the $S_1$ progeny for the regeneration trait by observing the regeneration results through the $S_2$ generation; and
e) growing the readily regenerable $S_1$ plants from the reproductive material. Where desirable or necessary, the agronomic characteristics of the target variety can be substantially preserved by expanding this method to include the further steps of repetitively:

a) backcrossing the readily regenerable progeny with plants from the target variety; and
b) selecting for expression of regenerability (or an associated marker gene) among the progeny of the backcross, until the desired percentage of the characteristics of the target variety are present in the progeny along with the trait for regenerability.

**Method of Use**

Seeds of the highly regenerable variety are surface sterilized and rinsed in sterile water. The seeds are then germinated and grown on a nutrient medium, preferably Murashige and Skoog basal medium in a light/dark cycle, preferably 16 hours of light to 8 hours of darkness. Hypocotyls from the seedlings are shredded and incubated in a mixed enzyme solution. Preferably such solution contains 0.2% cellulase, 0.2% macerase, and a 0.2% mixture of cellulase and xylanase, in S medium. Examples of the enzymes used are Cellulase R-10 Onozuka, Macerozyme Onozuka (both available from Yakult Pharmaceutical, Japan) and Driselase (Sigma). The macerated hypocotyls are filtered and the protoplasts pelleted through centrifugation. The protoplasts are then freed of cell debris by resuspension in medium preferably S medium containing 12% of a sucrose polymer, such as Ficoll, and centrifugation. In a preferred embodiment the protoplasts are centrifuged for 15 minutes at 250g. The protoplasts are then rinsed with any appropriate medium preferably S medium and resuspended in a culture medium. In a highly preferred embodiment the culture medium would be L4 medium modified to contain 1000 mg/l casamino acids and 0.1 mg/l 2,4-D. The protoplasts are then mixed with molten agarose in the culture medium. In a preferred embodiment the ratio of protoplasts to 6% (w/v) agarose would be 1:1 (v/v). The protoplast suspension is then dispensed into chilled petri dishes and incubated. The preferred protoplast density is 50,000 protoplasts/ml dispensed as 0.25 ml beads at a rate of 20 beads/petri dish. After incubation the protoplasts are surrounded with a culture medium, preferably L4, and the dishes are sealed and incubated in the dark at a suitable incubation temperature, preferably 25°C.

After an appropriate growth period, the cultures are placed in the light. At regular intervals old medium is removed from the cultures and replaced in with fresh culture medium. The preferred method is to remove the cultures from the dark after 10 days and place them in the light (30μ Einstein/m²/s). Every 8 to 10 days, 2.5 ml of the old medium is removed and replaced by fresh L4M medium modified by the adjustment of alpha-naphtaleneacetic acid (NAA) to 0.1 mg/l mannitol to 4% and sucrose to 0.1% (L'4M). The resulting calli are transferred to a regeneration medium such as R501 and maintained in the same conditions of light and temperature.

The regenerated shoots are transferred to a second regeneration medium such as R539 for elongation. Rooting is induced in excised shoots by dipping in 1 mg/ml indoleacetic acid (IAA) and in 0.1M KOH and continued subculture on the regeneration medium. Once the plants are well rooted, they are potted in soil mixture and hardened in a mist chamber prior to transfer to a greenhouse. Seed is collected from the fertile plants approximately 7 months from the first culturing of the protoplasts.

The composition of the various media used in the culturing and regeneration of the protoplasts are given in the following table.

## Media Composition

| Medium base | S | L4M | L'4M | R501 | R539 |
|---|---|---|---|---|---|
| SALTS g/l | | | | | |
| $CaCl_2 \cdot 2H_2O$ | 2 | 0.44 | 0.44 | 0.44 | 0.755 |
| KCl | 25 | 1.177 | 1.177 | | |
| | | | | | |
| SALTS mg/l | | | | | |
| $KH_2PO_4$ | | 68 | 68 | 170 | 1250 |
| $KNO_3$ | | | | 1900 | |
| $MgSO_4 \cdot 7H_2O$ | | 738 | 738 | 370 | 97.5 |
| $NH_4NO_3$ | | | | 1650 | |
| $(NH_4)HPO_4$ | | | | | 150 |
| $CoCl_2 \cdot 6H_2O$ | | 0.024 | 0.024 | 0.025 | 0.05 |
| $CuSO_4 \cdot 5H_2O$ | | 0.0025 | 0.0025 | 0.025 | 0.156 |
| $Na_2EDTA$ | | 37.25 | 37.25 | 37.25 | 10 |
| $FeSO_4 \cdot 7H_2O$ | | 27.85 | 27.85 | 27.85 | 7.5 |
| $H_3BO_3$ | | 6.2 | 6.2 | 6.2 | 2.5 |
| KI | | | | 0.83 | 0.5 |
| $MnSO_4 \cdot H_2O$ | | 0.17 | 0.17 | 16.9 | 5 |
| $Na_2MoO_4 \cdot 2H_2O$ | | 0.024 | 0.024 | 0.25 | 0.05 |
| $ZnSO_4 \cdot 7H_2O$ | | 0.28 | 0.28 | 8.6 | 0.5 |

| VITAMINS mg/l | | | | | |
|---|---|---|---|---|---|
| Biotin | | 0.01 | 0.01 | | |
| i-Inositol | | 100 | 100 | 100 | 100 |
| Glycine | | | | 2 | 2 |
| Nicotinic acid | 1 | 1 | 0.5 | 0.5 | |
| Ca pantothenate | 1 | 1 | | | |
| Pyridoxine-HCI | 1 | 1 | 0.5 | 0.5 | |
| Thiamine-HCI | | 1 | 1 | 0.4 | 0.4 |
| | | | | | |
| ORGANICS g/l | | | | | |
| L-Glutamine | | 1.095 | 1.095 | | |
| Casamino acids | 1 | 1 | 0.5 | 0.5 | |
| | | | | | |
| SUGARS g/l | | | | | |
| Sucrose | | 20 | 0.1 | 30 | |
| Mannitol | | 80 | 40 | | |
| | | | | | |
| HORMONES mg/l | | | | | |
| NAA | | 3 | 0.1 | 1 | |
| 2,4-D | | 0.1 | 0.1 | | |
| BAP | | 1 | 1 | 1 | |
| GA | | | | 0.1 | |
| | | | | | |
| Gel-rite g/l | | | | | 2 |
| Phytagar g/l | | | | 8 | |
| MES mg/l | 700 | 700 | 700 | | |
| pH | 5.6 | 5.7 | 5.7 | 5.6 | 5.5 |

## Deposits

A deposit of the seed of Variety PTO24, USDA Germplasm release SFM3, and of sunflower cultivar HA89 is maintained by Pioneer Hi-Bred International, Inc., 700 Capital Square, 400 Locust Street, Des Moines, Iowa 50309. Access to these deposits will be available during the pendency of this application to persons determined by the Commissioner of Patents and Trademarks to be entitled thereto under 37 CFR 1.14 and 35 USC 122. Upon allowance of any claims in this application, all restrictions on access required to be removed will be removed by affording access to one or more deposits of the same seed with the American Type Culture Collection, Rockville, MD.

## Claims

1. A sunflower plant having a frequency of regeneration of fertile plants of from about 80% to 100% from hypocotyl explants and the ability to regenerate fertile plants from hypocotyl protoplasts in tissue culture.

2. A sunflower plant having a genotype of which at least about 50% is a genotype out of sunflower release SFM3; a frequency of regeneration to whole fertile plants from hypocotyl explants of from about 80% to about 100%; and the ability to regenerate whole fertile plants from hypocotyl protoplasts.

3. A sunflower plant which is a second generation self from the 1-tall-2 selection from a fourth generation self of a male fertile segregant from a cross of cytoplasmic male sterile HA89 with pollen from <u>Helianthus</u> <u>petiolaris</u> and backcrossed once to cytoplasmic male sterile line HA89; having a frequency of regeneration to whole fertile plants from hypocotyl explants in tissue culture of from about 80% to about 100%; and the ability to regenerate whole fertile plants from hypocotyl protoplasts.

4. A plant part from the plant of Claim 1, selected from the group consisting of leaves, pollen, embryos, protoplasts, meristematic cells, hypocotyls, petals, seeds, stalks and tissue cultures thereof.

5. A sunflower plant regenerated from the tissue culture of Claim 4 and its plant parts.

6. Inbred sunflower seed according to Claim 5, designated PT024.

7. An $F_1$ hybrid sunflower plant and seed thereof produced by crossing an inbred sunflower plant according to Claim 1 with another, different sunflower plant and having a genotype one-half of which is substantially the genotype of the sunflower plant of Claim 1.

8. A method of regenerating fertile sunflower plants from isolated protoplasts comprising :
    a) sterilizing sunflower seeds according to Claim 4;
    b) germinating the seeds in a germination nutrient medium to form seedlings having hypocotyls;
    c) shredding the hypocotyls and macerating them in an enzyme solution comprising cellulase, macerase and xylanase to yield a mixture comprising hypocotyl protoplasts;
    d) isolating the protoplasts from the macerated hypocotyls;
    e) culturing the protoplasts on a tissue culture medium in the presence of callus-inducing plant hormones to form calli;
    f) toughening the calli by gradually lowering the hormone levels in the culture medium;
    g) regenerating shoots from the calli by transferring the calli successively to a shoot initiation medium and a shoot elongation medium;
    h) inducing rooting in the regenerated shoots by treating them with a rooting medium;
    i) growing the rooted shoots into plantlets on a fifth nutrient medium; and
    j) placing the rooted plantlets in soil.

9. A method of producing sunflower plants having high regeneration frequency from hypocotyl explants and regeneration ability from isolated protoplasts from a sunflower variety or population that has low or no regeneration ability, comprising the steps of:
    a) sexually crossing a plant from the variety or population with a plant according to Claim 1;
    b) recovering reproductive material produced as a result of the cross; and
    c) growing regenerable plants from the reproductive material.

10. A method according to Claim 9 for imparting regeneration capability in the sunflower variety that has no or poor regeneration capability which comprises the further steps of repetitively:
    a) backcrossing the regenerable plants produced with a plant from the non-regenerable variety; and
    b) selecting for expression of both regenerability and the other characteristics of the non-regenerable variety among the progeny of the backcrosses,
        until the desired percentage of the characteristics of the non-regenerable variety are present in the progeny along with regenerability from tissue culture and isolated protoplasts.

# PARTIAL EUROPEAN SEARCH REPORT

**European Patent Office**

which under Rule 45 of the European Patent Convention shall be considered, for the purposes of subsequent proceedings, as the European search report

Application Number

EP 91 31 1403

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,D | PLANT SCIENCE, vol. 42, 1985, pages 125-132, IE; K.E. PATERSON et al.: "Regeneration of Helianthus annuus inbred plants from callus" * Whole document * | 14,5,7-9 | A 01 H  4/00<br>A 01 H  5/10 |
| A | IDEM | 2,3 | |
| Y,D | PLANT SCIENCE, vol. 56, 1988, pages 177-181, IE; M. FREYSSINET et al.: "Fertile plant regeneration from sunflower (Helianthus annuus L.) immature embryos" * Whole document * | 1-5,7-10 | |
| Y,D | THEORETICAL AND APPLIED GENETICS, vol. 58, 1980, pages 265-271; G.J. KEYES et al.: "Genetic variation in tissue cultures of red clover" * Page 266, left-hand column, line 13 - page 270, right-hand column, line 3 * | 1-5,7-10 | |

-/-

**TECHNICAL FIELDS SEARCHED (Int. Cl.5)**

A 01 H

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :  1-5 and 7-10

Claims searched incompletely :

Claims not searched :  6 (plantvariety; art. 53b)

Reason for the limitation of the search:

| Place of search | Date of completion of the search | Examiner |
|---|---|---|
| THE HAGUE | 27-03-1992 | DISSEN H.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

## PARTIAL EUROPEAN SEARCH REPORT

Application Number

EP  91 31 1403

| DOCUMENTS CONSIDERED TO BE RELEVANT | | | CLASSIFICATION OF THE APPLICATION (Int. Cl.5) |
|---|---|---|---|
| Category | Citation of document with indication, where appropriate, of relevant passages | Relevant to claim | |
| A | Y.P.S. BAJAJ: "Biotechnology in agriculture and forestry", vol. 10; "Legumes and oilseed crops I", 1990, pages 434-452, Springer-Verlag, Berlin, DE, Chapter IV.4: K.E.P. ROBINSON et al.: "Sunflower (Helianthus annuus L.): Establishment of cultures, transformation and the regeneration of plants" * Page 436, line 32 - page 444, line 3; table 3 * --- | 1-5,8,9 | |
| A | EP-A-0 266 287 (RHONE-POULENC AGROCHIMIE) * Page 2, line 39 - page 6, line 38 * --- | 1-5,7,8 | |
| A | US-A-4 687 743 (PATERSON et al.) * Column 3, line 29 - column 12, line 27 * --- | 1-5,8,9 | TECHNICAL FIELDS SEARCHED (Int. Cl.5) |
| A,D | ACTA BOTANICA NEERLANDICA, vol. 38, no. 1, March 1989, pages 1-23; S. ROEST et al.: "Plant regeneration from protoplasts: a literature review" * Page 10, line 23 - page 12, line 5; table 1 * --- | 1-5,8,9 | |
| A,D | ZEITSCHRIFT FÜR PFLANZENPHYSIOLOGIE, vol. 101, 1981, pages 119-130; H. BINDING et al.: "Comparative studies on protoplast regeneraation in herbaceous species of the dicotyledoneae class" * Whole document * ----- | 1,2,4,5 ,8 | |

EPO FORM 1503 03.82 (P0410)